# EUROPEAN PATENT SPECIFICATION

(11) **EP 2 004 241 B1**
(45) Date of publication and mention of the grant of the patent: **07.08.2013**
(21) Application number: 07736139.2
(22) Date of filing: 28.03.2007
(51) Int. Cl.: A61K 49/00, A61B 5/00, A01N 63/00, A61F 13/00, C12N 5/00, A01N 1/00

(54) **IMPLANTABLE SENSOR**
IMPLANTIERBARER SENSOR
CAPTEUR IMPLANTABLE

(30) Priority: 28.03.2006 US 786532 P
(43) Date of publication of application: 24.12.2008
(73) Proprietor: GLUSENSE LTD., 71700 Modi'in (IL)
(72) Inventor: GROSS, Yossi, 73160 Moshav Mazor (IL); HYMAN, Tehila, 71700 Modi'in (IL)
(74) Representative: Evens, Paul Jonathan
(86) International application number: PCT/IL2007/000399
(87) International publication number: WO 2007/110867

(56) References cited:
- WO-A1-2004/089465
- WO-A2-2006/006166
- US-A1- 2002 016 535
- DATABASE MEDLINE [Online] FEHR M. ET AL.: 'Minimally invasive dynamic imaging of ions and metabolites in living cells', XP008138532 Database accession no. (15134757) & CURR. OPIN. PLANT BIOL. vol. 7, no. 3, June 2004, pages 345 - 351
- FEHR M. ET AL.: 'In vivo imaging of the dynamics of glucose uptake in the cytosol of COS-7 cells by fluorescent nanosensors' J. BIOL. CHEM. vol. 278, no. 21, 23 May 2003, pages 19127 - 19133, XP002553883
- DATABASE MEDLINE [Online] LAXMAN B. ET AL.: 'Noninvasive real-time imaging of apoptosis', XP008138515 Database accession no. (12475931) & PROC. NATL. ACAD. SCI. USA vol. 99, no. 26, 24 December 2002, pages 16551 - 16555
- DATABASE MEDLINE [Online] SERGANOVA I. ET AL.: 'Reporter gene imaging: potential impact on therapy', XP008138526 Database accession no. (16243653) & NUCL. MED. BIOL. vol. 32, no. 7, October 2005, pages 763 - 780
- DATABASE MEDLINE [Online] DEUSCHLE K. ET AL.: 'Genetically encoded sensors for metabolites', XP008138528 Database accession no. (15688353) & CYTOMETRY A vol. 64, no. 1, March 2005, pages 3 - 9
- DATABASE MEDLINE [Online] PICKUP J.C. ET AL.: 'In vivo glucose monitoring: the clinical reality and the promise', XP008138530 Database accession no. (15741056) & BIOSENS. BIOELECTRON. vol. 20, no. 10, 15 April 2005, pages 1897 - 1902

## Description

### FIELD OF THE INVENTION

The present invention relates generally to implantable sensors, and specifically to and apparatus for sensing blood glucose concentrations.

### BACKGROUND OF THE INVENTION

Fluorescence resonance energy transfer (FRET) involves the transfer of photon energy from an excited fluorophore (the donor) to another fluorophore (the acceptor), when the donor and acceptor molecules are in close proximity to each other. FRET enables the determination of the relative proximity of the molecules, for investigating, for example, molecular interactions between two protein partners, structural changes within one molecule, and ion concentrations. Fluorescent proteins (FPs) can be genetically fused to proteins of interest and expressed in cells. FP pairs useful for performing FRET measurements in living cells include cyan fluorescent protein (CFP) as the donor, and yellow fluorescent protein (YFP) as the acceptor, because the emission spectrum of CFP partially overlaps the excitation spectrum of YFP.

US Patent 3,837,339 to Aisenberg et al., describes techniques for monitoring blood glucose levels, including an implantable glucose diffusion-limited fuel cell. The output current of the fuel cell is proportional to the glucose concentration of the body fluid electrolyte and is therefore directly indicative of the blood glucose level. This information is telemetered to an external receiver which generates an alarm signal whenever the fuel cell output current exceeds or falls below a predetermined current magnitude which represents a normal blood glucose level. Valve means are actuated in response to the telemetered information to supply glucose or insulin to the monitored living body.

US Patents 5,368,028 and 5,101,814 to Palti describe methods and apparatus for monitoring blood glucose levels by implanting glucose sensitive living cells, which are enclosed in a membrane permeable to glucose but impermeable to immune system cells, inside a patient. Cells that produce detectable electrical activity in response to changes in blood glucose levels are used in the apparatus along with sensors for detecting the electrical signals, as a means for detecting blood glucose levels. Human beta cells from the islets of Langerhans of the pancreas, sensor cells in taste buds, and alpha cells from the pancreas are discussed as appropriate glucose sensitive cells.

US Patents 6,091,974 and 5,529,066 to Palti, describe a capsule for encapsulating implantable cells for improving the detectability of electrical signals generated by the cells. The capsule includes a low-conductivity (high electrical resistance) membrane and a semi-permeable (low electrical resistance) membrane. The low-conductivity membrane seals around the circumference of the cell mass between the electrical poles of the capsule, and further extends for increasing the electrical resistance between the poles. The semi-permeable membrane enables nutrients and waste materials to flow to and from the cell mass. The semi-permeable membrane encloses at least one of the poles of the cell mass, and cooperates with the low-conductivity membrane to completely enclose the cell mass. The low-conductivity membrane may enclose one of the poles, if desired. Electrodes are used to detect the electrical signals from the cell mass.

US Patent Application 2002/0038083 to Houben and Larik describes methods and apparatus for monitoring blood glucose levels by implanting glucose sensitive living cells, which are enclosed in a membrane permeable to glucose but impermeable to immune system cells, inside a patient. The living cells come from the islets of Langerhans of the pancreas and have been genetically engineered so as to grow on a substrate containing interdigitated electrodes, which serves as a sensor of cellular electrical activity.

US Patent 5,443,508 to Giampapa describes an implantable biological agent delivery system. The system includes a pod adapted for subcutaneous implantation beneath the dermis of the skin. The pod includes a porous surface and has at least one internal chamber which is in fluid communication with the porous surface. The system includes a dome adapted to be detachably secured to the chamber. The dome includes interior chambers, each in fluid communication with the interior of the pod. Prior to implantation, the chambers are loaded with bioactive agents, such as hormones, enzymes, biologic response modifiers, free radical scavengers, or genetically altered cell cultures. Time-release micropumps pump the agents into the interior chambers of the pod for transmission through the porous surfaces into a growth factor-stimulated capillary matrix and then to the bloodstream of the subject. The pod may be removed, refilled, and resecured to the dome upon exhaustion of its contents or upon medical requirement for changes in medication, or may be percutaneously refilled in situ through injection into the dome. The surface of the pod may be treated with one or more vascular growth factors or related biologic molecules.

US Patent 5,614,378 to Yang et al. describes a photobioreactor system for oxygen production for a closed ecological life support system. The photobioreactor is described, among other things, as being useful for converting carbon dioxide to oxygen in an artificial lung.

US Patent 5,855,613 to Antanavich et al. describes embedding cells in a thin sheet of alginate gel that is then implanted in a body.

US Patent 5,834,005 to Usala describes immunoisolating cells by placing them in a chamber that is implanted inside the body. In the chamber, the cells are shielded from the immune system by means of a membrane permeable to small molecules such as glucose, oxygen, and the hormone secreted by the cells, but impermeable to cells and antibodies.

US Patent 4,402,694 to Ash et al. describes a body cavity access device for supplying a hormone to a patient. The device includes an implantable housing placed in the body and having an impermeable extracorporeal segment and a semipermeable subcutaneous segment. A hormone source such as live, hormone-producing cells, e.g., pancreatic islet cells, is then removably positioned in the housing to provide a hormone supply to the patient. A sensor can be located within the subcutaneous segment and operably associated with a dispenser to release medication into the housing and to the patient.

US Patent 5,011,472 to Aebischer et al. describes techniques for providing hybrid, modular systems for the constitutive delivery of active factor to a subject and, in some instances, to specific anatomical regions of the subject. The systems include a cell reservoir containing living cells capable of secreting an active agent, which is preferably adapted for implantation within the body of the subject and further includes at least one semipermeable membrane, whereby the transplanted cells can be nourished by nutrients transported across the membrane while at the same time protected from immunological, bacterial, and viral assault. The systems further include a pumping means, which can be implantable or extracorporeal, for drawing a body fluid from the subject into the cell reservoir and for actively transporting the secreted biological factors from the cell reservoir to a selected region of the subject.

US Patent 5,116,494 to Chick et al. describes a device that serves as an artificial pancreas. The device comprises a hollow fiber which is surrounded by islets of Langerhans enclosed in a housing. The islets are suspended in a temperature sensitive matrix which is sufficiently viscous to support islets at a temperature below about 45 degrees C and sufficiently fluid to enable removal of islet suspension at a temperature above about 45 degrees C. A warm (e.g., 48 degree to 50 degree C solution) may be flushed through the device to change the physical state of the temperature sensitive matrix from a semi-solid state to a liquefied semi-gel state. The temperature sensitive supporting material is described as enabling long-term maintenance of islet cells in in vitro culture.

US Patent 5,741,334 to Mullon et al. describes an artificial pancreatic perfusion device comprising a hollow fiber having a porosity ranging from about 25 Kd to about 200 Kd. The hollow fiber has one end connected to a blood vessel for receiving blood and a second end connected to a blood vessel for returning the blood. Islets of Langerhans surround the hollow fiber. The hollow fiber and islets are surrounded by a housing comprising a semipermeable membrane having a pore size small enough to offer protection to the islets and host from immune reactive substances.

US Patent 5,702,444 to Struthers et al. describes an implantable artificial endocrine pancreas comprising a reactive body of soft, plastic, biocompatible, porous hydratable material supporting a multiplicity of endocrine pancreatic islets in isolated spaced relationship from each other, and a microporous barrier membrane enveloping and supporting the body, in spaced relationship from the pancreatic islets therein and through which molecules having a molecular weight greater than 60,000 Daltons cannot penetrate.

US Patent 6,630,154 to Fraker et al. describes a composition including at least one glycosaminoglycan, e.g., CIS, at least one perfluorinated substance and at least one alginate, e.g., sodium alginate.

PCT Publication WO 90/15526 to Kertz describes an integument and related process for the culturing and growing of living organic material. The integument includes a cellule made of a gas-permeable, liquid- and contaminant-impermeable membrane for completely enclosing and sealing the culture from biological contaminants in the ambient environment. The membrane allows gas exchange between the living organic material and the ambient environment to provide enhanced growth and the prevention of contamination.

PCT Publication WO 01/50983 to Vardi et al., and US Patent Application 10/466,069 in the national phase thereof describe an implantable device comprising a chamber for holding functional cells and an oxygen generator for providing oxygen to the functional cells. In one embodiment, the oxygen generator comprises photosynthetic cells that convert carbon dioxide to oxygen when illuminated. In another embodiment, the oxygen generator comprises electrodes that produce oxygen by electrolysis. In another embodiment, an implantable chamber is used as part of a system for detecting or monitoring the level of a substance in body fluids. Such a system includes a detector adapted to monitor a property of the functional cells that is correlated with the level of the substance in the medium surrounding the functional cells.

US Patent 6,197,534 to Lakowicz by describes methods and sensors for detecting the presence or concentration of an analyte in a sample, preferably a sugar such as glucose, which preferably utilizes a labeled engineered protein, or fragment thereof, that is capable of binding the analyte to be detected.

US Patent 5,246,867 to Lakowicz by describes a method for measuring the concentration of a saccharide, conjugated saccharide or polysaccharide of interest using luminescent lifetimes and energy transfer in which an energy transfer donor-acceptor pair is added to a sample to be analyzed, the donor of the donor-acceptor pair being photoluminescent. The acceptor is bound to a carrier, while the donor and any saccharide, conjugated saccharide or polysaccharide of interest present in the sample compete for binding sites on the carrier. The sample is irradiated and the resultant emission detected. Energy transfer occurs between the donors and the acceptors, which produces a detectable lifetime change of the fluorescence of the donor. The lifetime change is reduced or even eliminated by the competitive binding of a saccharide, conjugated saccharide or polysaccharide of interest to the donor. By measuring the apparent luminescent lifetime, the amount of a saccharide, conjugated saccharide or polysaccharide of interest in the sample can be determined.

Methods for immunoprotection of biological materials by encapsulation are described, for example, in US Patents 4,352,883, 5,427,935, 5,879,709, 5,902,745, and 5,912,005. The encapsulating material is typically selected so as to be biocompatible and to allow diffusion of small molecules between the cells of the environment while shielding the cells from immunoglobulins and cells of the immune system. Encapsulated beta cells, for example, can be injected into a vein (in which case they will eventually become lodged in the liver) or embedded under the skin, in the abdominal cavity, or in other locations. Fibrotic overgrowth around the implanted cells, however, gradually impairs substance exchange between the cells and their environment. Hypoxia of the cells typically leads to cell death.

PCT Patent Publication WO 01/50983 to Bloch et al. describes methods and apparatus for monitoring physiological variables in a living organism by implanting, inside a patient, functional tissue or cells, which are enclosed in a membrane permeable to glucose and other nutrients but impermeable to immune system cells. In order to maintain a sufficient oxygen supply for the functional cells an oxygen generator comprising photosynthetic cells and a light source is placed inside the membrane. In an application described in the '983 publication, an implantable chamber is used as part of a system for detecting or monitoring the level of a substance in body fluids. Such a system includes a detector adapted to monitor a property of the functional cells that is correlated with the level of the substance in the medium surrounding the functional cells.

PCT Patent Publication WO 2006/006166 to Gross et al., describes a protein, including a glucose binding site, cyan fluorescent protein (CFP), and yellow fluorescent protein (YFP). The protein is described as being configured such that binding of glucose to the glucose binding site causes a reduction in a distance between the CFP and the YFP. Apparatus is also described for detecting a concentration of a substance in a subject, the apparatus comprising a housing adapted to be implanted in the subject. The housing comprises an optical detector, and cells genetically engineered to produce, in a patient's body, a FRET protein comprising a fluorescent protein donor, a fluorescent protein acceptor, and a protein containing a binding site for the substance.

United States Patent Application Publication 2005/0118726 to Schultz et al. describes a method for making a fusion protein, having a first binding moiety having a binding domain specific for a class of analytes that undergoes a reproducible allosteric change in conformation when said analytes are reversibly bound; a second moiety and third moiety that are covalently linked to either side of the first binding moiety such that the second and third moieties undergo a change in relative position when an analyte of interest molecule binds to the binding moiety; and the second and third moieties undergo a change in optical properties when their relative positions change and that change can be monitored remotely by optical means. A system and method is also described for detecting glucose that uses such a fusion protein in a variety of formats including a subcutaneously and in a bioreactor.

United States Patent Application Publication 2003/0232370 to Trifiro describes a glucokinase protein in which the catalytic activity has been disabled in order to enable its use as a glucose sensor. The catalytically disabled glucokinase protein is described as being used as the glucose sensor in hand-held glucose monitors and in implantable glucose monitoring devices. The glucose sensor is described as being incorporated into biomedical devices for the continuous monitoring of glucose and administration of insulin.

US Patent 5,998,204 to Tsien et al. describes fluorescent protein sensors for detection of analytes. Fluorescent indicators including a binding protein moiety, a donor fluorescent protein moiety, and an acceptor fluorescent protein moiety are described. The binding protein moiety has an analyte-binding region which binds an analyte and causes the indicator to change conformation upon exposure to the analyte. The donor moiety and the acceptor moiety change position relative to each other when the analyte binds to the analyte-binding region. The donor moiety and the acceptor moiety exhibit fluorescence resonance energy transfer when the donor moiety is excited and the distance between the donor moiety and the acceptor moiety is small. The indicators can be used to measure analyte concentrations in samples, such as calcium ion concentrations in cells.

United States Patent Application Publication 2003/0134346 to Amiss et al. describes compositions of mutated binding proteins containing reporter groups, analyte biosensor devices derived therefrom, and their use as analyte biosensors both in vitro and in vivo.

US Patent 5,089,697 to Prohaska describes a fiber optic sensing device for use in a variety of different applications. The fiber optic sensing device includes a light source and a first fiber optic waveguide for receiving light from the light source. A second fiber optic waveguide is provided for receiving light from the first fiber optic waveguide. The fiber optic sensing device further includes means mounting the first and second fiber optic waveguides for relative movement to vary the light transmitted between the first and second fiber optic waveguides. A sensor is provided for sensing the light transmitted between the first and second fiber optic waveguides.

US Patents 4,981,779 and 5,001,054 to Wagner describes methods for monitoring the glucose level in a body fluid. Apparatus described includes a conjugate of glucose oxidase and a fluorescent dye coated onto an optical fiber in contact with the body fluid, a source of excitation light and a fluorescence emission detector. Glucose is oxidized by oxygen in the body fluid, causing a decrease in oxygen concentration at the enzyme. The fluorescent dye is sensitive to oxygen quenching so that, when the oxygen concentration decreases, fluorescence emission increases in direct proportion to the glucose concentration in the fluid.

US Patent Application Publication 2005/0136092 to Rotem et al, describes apparatus including a chamber, which is adapted to be implanted in a body of an individual, the chamber including functional cells and chlorophyll-containing elements comprising chlorophyll of an obligate photoautotroph.

In an article by Klueh U. et al., entitled, "Enhancement of implantable glucose sensor function in vivo using gene transfer-induced neovascularization," Biomaterials, April, 2005, 26(10): 155-63 it is stated that the in vivo failure of implantable glucose sensors is thought to be largely the result of inflammation and fibrosis-induced vessel regression at sites of sensor implantation. To determine whether increased vessel density at sites of sensor implantation would enhance sensor function, cells genetically engineered to over-express the angiogenic factor (AF) vascular endothelial cell growth factor (VEGF) were incorporated into an ex ova chicken embryo chorioallantoic membrane (CAM)-glucose sensor model. The VEGF-producing cells were delivered to sites of glucose sensor implantation on the CAM using a tissue-interactive fibrin bio-hydrogel as a cell support and activation matrix. This VEGF-cell-fibrin system induced significant neovascularization surrounding the implanted sensor, and significantly enhanced the glucose sensor function in vivo.

The following references may be of interest:
Ye K et al., "Genetic engineering of an allosterically based glucose indicator protein for continuous glucose monitoring by fluorescence resonance energy transfer," Analytical Chemistry, 2003, 75(14), 3451-3459
Tolosa L et al., "Glucose sensor for low-cost lifetime-based sensing using a genetically engineered protein," Analytical Biochemistry, 267, 114-120 (1999)
Hellinga H et al., "Protein engineering and the development of generic biosensors," Tibtech, Volume 16 (April, 1998)
Fehr M et al., "In vivo imaging of the dynamics of glucose uptake in the cytosol of COS-7 cells by fluorescent nanosensors," J. Biol. Chem., 278(21):19127-19133 (2003)
Ackland-Berglund, C et al., "Efficacy of tetracycline-controlled gene expression is influenced by cell type," BioTechniques 18, 196-200 (1995)
Fillat C et al., "Suicide gene therapy mediated by the herpes simplex virus thymidine kinase gene / ganciclovir system: Fifteen years of application," Current Gene Therapy, 3(1), pp. 13-26, (February, 2003)
Scognamiglio V et al., "Protein-based biosensors for diabetic patients," Journal of Fluorescence, 14(5), 491-498 (September, 2004)
Moschou E et al., "Fluorescence glucose detection: Advances toward the ideal in vivo biosensor," Journal of Fluorescence, 14(5), 535-547 (September, 2004)
Reszka R et al., "Liposome-mediated suicide gene therapy in humans," Methods in Enzymology, 391, 200-208 (2005)
Deuschle K et al., "Construction and optimization of a family of genetically encoded metabolite sensors by semirational protein engineering," Protein Sci. 14: 2304-2314 (2005)

Diabetes is a disorder that affects many people and results from the inability of the body to properly utilize and metabolize carbohydrates, particularly glucose. Normally the balance between glucose in the blood and glucose in body tissue cells is maintained by insulin, a hormone produced by the pancreas that controls the transfer of glucose from the blood into body tissue cells. Abnormal levels of glucose in the blood cause many complications and pathologies, leading to premature death in many cases.

Abnormally high levels of blood glucose can be controlled in many cases by the injection of insulin into the body. The amount of insulin to be injected depends upon the level of glucose in the blood, leading to a demand for accurate blood glucose sensors. Since regular monitoring of blood glucose levels allows for better regulation via insulin injections, it is desirable to have a simple and convenient means for monitoring blood glucose levels. Historically, the most common method to determine blood glucose levels was to obtain a small blood sample by piercing the finger and then placing the blood in an analyzer.

To avoid the regular piercing of a finger and to obtain more continuous monitoring of blood glucose levels, implantable glucose sensors have been described. In some cases, implantable sensors have been described that include cells such as transplanted pancreatic cells. These pancreatic cells are described as responding in a manner proportional to blood glucose levels, such that by monitoring the cellular response a blood glucose level can be determined.

### SUMMARY OF THE INVENTION

A device according to the invention is defined in claim 1.

In some embodiments, genetically-engineered living cells are implanted in a patient. The cells are engineered to produce a protein that is able to bind with an analyte and to undergo a conformational change in a detectable manner. An implanted device detects the conformational change, and, in response generates a signal indicative of a level of the analyte in the patient. Typically, but not necessarily, FRET techniques known in the art are used to detect the conformational change.

There is therefore provided a method, including:
implanting in a human subject engineered live cells that produce a molecule; and
determining a physiological parameter of the subject by sensing from within the subject a characteristic of the molecule.
There is also provided a method, including:
implanting in a human subject engineered live cells that produce a protein; and
determining a physiological parameter of the subject by sensing from within the subject a characteristic of the protein.

In an embodiment, the cells express the protein and direct the protein to cell membranes of the cells.

In an embodiment, the cells secrete the protein.

In an embodiment, the parameter includes a glucose level of the subject, and determining the parameter includes determining the parameter in a continuous manner.

In an embodiment, the engineered live cells produce two proteins, having different respective affinities for an analyte, and determining the physiological parameter includes determining an extent of binding of the analyte to the first and second proteins.

In an embodiment, determining the parameter includes determining an indication of a blood glucose level of the subject.

In an embodiment, the cells produce a protein, wherein determining the parameter includes determining an extent of binding of an analyte to the protein, and the method includes calibrating the determining of the extent of binding by:
intermittently establishing a basal level of binding of the analyte to the protein by reducing binding of the analyte to the protein; and
measuring the basal level of binding.

In an embodiment, the analyte includes glucose, and measuring the basal level of binding includes measuring the level of binding of the glucose to the protein.

In an embodiment, reducing the binding includes electrochemically reducing the binding.

In an embodiment, reducing the binding includes facilitating consumption of the analyte by algae.

In an embodiment, reducing the binding includes facilitating consumption of the analyte by the live engineered cells.

In an embodiment, reducing the binding includes facilitating consumption of the analyte by cells other than the live engineered cells.

In an embodiment, reducing the binding includes facilitating consumption of the analyte by a line of cells selected from the group consisting of: COS7 cells, and HepG2 cells.

There is additionally provided an apparatus, including:
an implantable chamber that includes engineered live cells that produce a molecule; and
an implantable sensor adapted to sense a characteristic of the molecule and to generate a signal, in response to the sensed characteristic, that is indicative of a physiological parameter of the subject.

There is yet additionally provided an apparatus, including:
an implantable chamber that includes engineered live cells that produce a protein; and
an implantable sensor adapted to sense a characteristic of the protein and to generate a signal, in response to the sensed characteristic, that is indicative of a physiological parameter of the subject.

In an embodiment, the engineered live cells produce two proteins, having different respective affinities for an analyte, and the implantable sensor is configured to determine the physiological parameter by determining an extent of binding of the analyte to the first and second proteins.

In an embodiment, the cells express the protein and direct the protein to cell membranes of the cells.

In an embodiment, the cells secrete the protein.

In an embodiment, the engineered live cells produce a glucose binding protein.

In an embodiment, the engineered live cells produce GLUT2.

In an embodiment, the sensor is adapted to generate a signal indicative of blood glucose level.

In an embodiment:
the cells produce a protein,
the sensor is configured to determine an extent of binding of an analyte to the protein,
the apparatus is configured to intermittently establish a basal level of binding of the analyte to the protein by reducing binding of the analyte to the protein, and
the sensor is configured to calibrate the determining of the extent of binding by measuring the basal level of binding.

In an embodiment, the analyte includes glucose, and wherein the sensor is configured to measure the level of binding of the glucose to the protein.

In an embodiment, the apparatus is configured to reduce the binding by electrochemically reducing the binding.

In an embodiment, the apparatus includes algae, configured to reduce the binding by consuming at least some of the analyte.

In an embodiment, the engineered live cells are disposed within the apparatus in a manner that facilitates consumption of the analyte by the live engineered cells.

In an embodiment, the apparatus includes cells other than the live engineered cells, and the apparatus is configured to reduce the binding by facilitating consumption of the analyte by the cells other than the live engineered cells.

In an embodiment, the apparatus includes a line of cells selected from the group consisting of: COS7 cells, and HepG2 cells, and the apparatus is configured to reduce the binding by facilitating consumption of the analyte by the line of cells selected from the group consisting of: COS7 cells, and HepG2 cells.

There is still additionally provided a cell capable of producing a sensor protein, the sensor protein including:
a glucose binding protein;
a cyan fluorescent protein (CFP); and
a yellow fluorescent protein (YFP),
wherein the sensor protein is configured such that binding of glucose to the glucose binding protein causes a change in a distance between the CFP and the YFP.

In an embodiment, the fluorescent proteins are disposed at respective ends of the glucose binding protein.

In an embodiment, at least one of the fluorescent proteins is disposed within the glucose binding protein.

In an embodiment, at least one of the proteins includes a mutated protein.

In an embodiment, at least one of the proteins includes a wild type protein.

In an embodiment, the sensor protein is encoded by an isolated nucleic acid fragment having a nucleotide sequence represented by Sequence No. 1.

In an embodiment, a biocompatible housing is provided that is adapted for implantation in a human body, the cell being disposed within the housing.

There is also provided an apparatus for detecting a concentration of an analyte in a subject, the apparatus including a housing adapted to be implanted in the subject,
the housing including an optical detector adapted to detect a level of fluorescence resonance energy transfer (FRET), and
the apparatus including live cells genetically engineered to produce, in a subject's body, a sensor protein including a fluorescent protein donor, a fluorescent protein acceptor, and a binding protein for the analyte.

In an embodiment, the housing includes a light source, a processor, and a transmitter, and wherein the cells are not disposed within the housing.

In an embodiment, the housing is adapted to be implanted at a first site of the subject, and wherein the apparatus is configured to facilitate implantation of the cells at a second site of the subject 3-20 cm from the first site.

In an embodiment, the cells are genetically engineered to secrete a factor selected from the group consisting of: an anti-fibrosis factor and an anti-inflammatory factor, and wherein the apparatus is configured to allow passage out of the apparatus of the selected factor.

In an embodiment, the cells are genetically engineered to secrete an angiogenic factor, and wherein the apparatus is configured to allow passage out of the apparatus of the angiogenic factor.

In an embodiment, the angiogenic factor includes vascular endothelial growth factor (VEGF).

In an embodiment, the apparatus includes a unit adapted for use external to a body of the subject, and adapted to receive from the optical detector a signal indicative of a blood glucose level of the subject.

In an embodiment, the apparatus includes a drug-delivery unit adapted to receive a signal indicative of a blood glucose level of the subject and to administer a drug in response to the signal.

In an embodiment, the apparatus includes an insulin pump which is adapted to administer insulin in response to the signal.

There is further provided an apparatus for detecting a concentration of an analyte in a subject, the apparatus including:
a first housing adapted to be implanted in the subject, the first housing including an optical detector adapted to detect a level of fluorescence resonance energy transfer (FRET); and
a second housing adapted to be implanted in the subject 3-20 cm from the first housing, the second housing including live cells genetically engineered to produce, in a patient's body, a sensor protein including a fluorescent protein donor, a fluorescent protein acceptor, and a binding protein for the analyte.

In an embodiment, the apparatus includes a set of one or more optical fibers coupling the first housing to the second housing.

There is still further provided a cell genetically engineered to secrete glucose oxidase (GOx).

In an embodiment, apparatus is provided that is adapted to amperometrically measure an indication of an oxygen level that varies in response to an interaction between the GOx and glucose in blood of a patient.

There is yet further provided, in accordance with an embodiment of the invention, a cell genetically engineered to secrete glucokinase.

There is also provided a method including:
implanting live cells in a subject; and
subsequently administering, to the subject, a drug capable of killing the cells.

In an embodiment, administering the drug includes administering a drug capable of activating a suicide gene of the cells.

There is additionally provided method including:
implanting live cells in a subject;
administering, to the subject, a promoter that regulates protein expression of the cells.

There is yet additionally provided a method including implanting a glucose sensor in cerebral spinal fluid (CSF) of a spinal cord of a subject.

There is still additionally provided a method including:
implanting an active medical device inside bone of a subject; and
detecting or affecting a property of blood in fluid communication with the medical device.

There is also provided a method including:
implanting, in a subject, live cells that are genetically engineered to express a promoter that is inducible by a substance; and
administering the substance to the subject.

The present invention will be more fully understood from the following detailed description of embodiments thereof, taken together with the drawings, in which:

### BRIEF DESCRIPTION OF THE DRAWINGS

Figs. 1, 2, and 3 are schematic illustrations of an implantable device for detecting a concentration of an analyte in a subject;
Fig. 4 is a schematic illustration of a system including the device of Fig. 2, in accordance with an embodiment of the present invention. In other embodiments (not shown), the system of Fig. 4 comprises the device of Fig. 1 or Fig. 3; and
Fig. 5 is a graph showing experimental in vitro obtained results using genetically-engineered live cells, in accordance with an embodiment of the present invention.

### DETAILED DESCRIPTION OF EMBODIMENTS

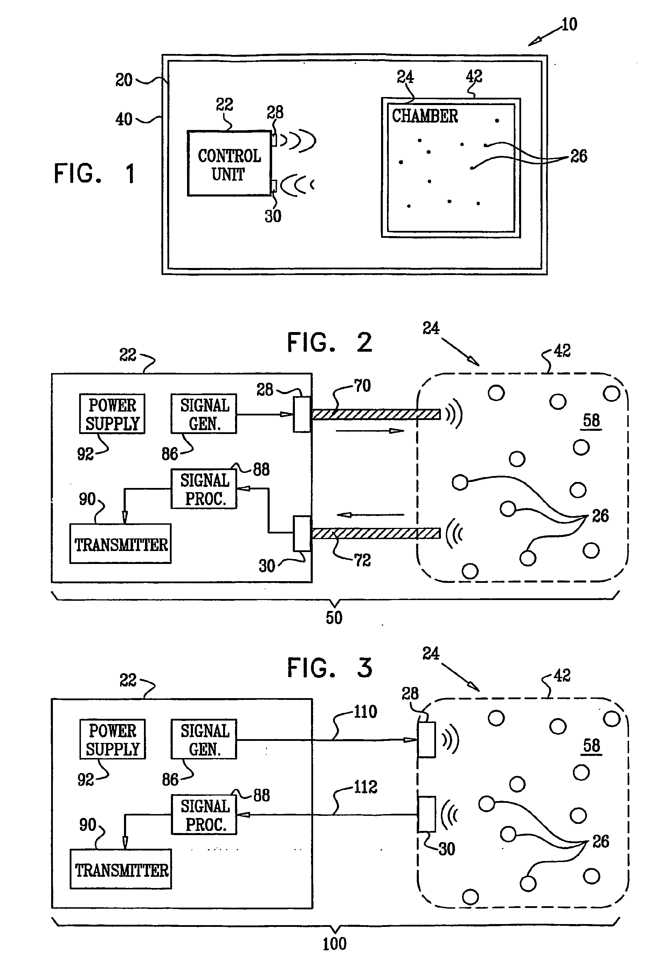

Fig. 1 is a schematic illustration of an implantable device 10 for detecting a concentration of an analyte in a subject, such as a blood constituent or other body fluid constituent of the subject, not in accordance with the present invention. Device 10 comprises an implantable housing 20 that holds a control unit 22 and cells 26. For some applications, no housing is provided. Control unit 22 comprises a light source 28, such as a laser or an LED, and an optical detector 30, which may comprise a CCD or other suitable detector. Cells 26 are genetically engineered to produce, in a patient's body, sensor proteins comprising a fluorescent protein donor, a fluorescent protein acceptor, and a binding protein for the analyte. As appropriate, the sensor proteins may generally reside in the cytoplasm of cells 26 and/or may be targeted to reside on the cell membranes of cells 26, and/or may be secreted by cells 26 into a chamber 24. The sensor proteins are configured such that binding of the analyte to the binding protein changes the conformation of the sensor proteins, and thus the distance between respective donors and acceptors. Using the signal from optical detector 30, control unit 22 detects this change in distance and determines the quantity of the signal resulting from subsets of the sensor protein that are in each of the two conformations, thereby enabling a calculation of the concentration of the analyte.

For some applications, chamber 24 holds cells 26 within a membrane 42. Alternatively, the cells are held directly in housing 20, which is surrounded by a membrane 40. For some applications, the cells are placed in a matrix (e.g., a hydrogel matrix), while for other applications, the cells are placed in suspension. Regardless of which membrane is used, the membrane is typically configured to allow passage only of molecules smaller than a threshold such as about 200 dalton, 50 kilodaltons, 100 kilodaltons, or 200 kilodaltons.

Fig. 2 is a schematic illustration of an implantable device 50 for detecting a concentration of an analyte in a subject, in accordance with an embodiment of the present invention. Control unit 22 comprises a power supply 92, which may comprise a long-life battery and/or be rechargeable, a signal generator 86, which drives light source 28, and a signal processor 88, which receives a signal from optical detector 30 and typically processes it in order to determine an indication of the concentration of the analyte. Chamber 24 typically comprises a gel matrix 58, in which cells 26 are embedded. In these respects, device 50 is similar to device 10 described hereinabove.

Device 50 typically comprises a set of optical fibers 70 coupled between light source 28 and chamber 24, through which the light source illuminates cells 26. A set of optical fibers 72 carries light from chamber 24 to signal processor 88. For some applications, a common set of optical fibers operate bidirectionally, carrying light from light source 28 to chamber 24, and carrying modified light from chamber 24 back to control unit 22. In any case, the distance between control unit 22 and chamber 24 is typically between about 3 and about 20 cm.

It is noted that although Fig. 2 shows optical fibers 70 and 72 coupling control unit 22 to chamber 24, other embodiments provide no optical fibers coupling the control unit to the chamber. In these embodiments, the proximity of light source 28 and optical detector 30 to chamber 24 is typically sufficient to generate a signal as described herein for determination of the level of the analyte.

Fig. 3 is a schematic illustration of an implantable device 100 for detecting a concentration of an analyte in a subject, not in accordance with present invention. Device 100 is generally similar to device 50, except as noted below. Light source 28 is coupled to (e.g., attached to a wall of) chamber 24, and electric leads 110 couple signal generator 86 to the light source. Alternatively or additionally, optical detector 30 is coupled to (e.g., attached to a wall of) chamber 24, and electric leads 112 couple signal processor 88 to the optical detector. In an embodiment, a plurality of optical detectors 30 are coupled to chamber 24, in order to enhance signal detection. For some applications, a combination of electric leads and in accordance with the invention optical fibers are used to couple control unit to chamber 24.

Reference is now made to Figs. 1, 2, and 3.

For some applications, devices 10, 50, or 100 incorporate optical detection, processing, transmitting, or other technologies described in one or more of above-cited US Patents 5,089,697, 5,001,054, or 4,981,779.

Depending on the desired frequency of measurements of the level of the analyte, light source 28 may be configured to operate in a steady-state mode, or to emit light in pulses (e.g., to facilitate one measurement every 3 minutes).

Typically, but not necessarily, cells 26 comprise slowly-dividing cells. Alternatively or additionally, cells 26 include but are not limited to at least some of the following: beta cells, neural cells (e.g., Neuro2A, PC12), fibroblast cells (e.g., BHK21, L929, HF-SKFII, NIH/3T3), liver cells, or CHO cells. Alternatively, cells 26 comprise other types of cells.

For some applications, the analyte includes glucose, and the binding protein is a wild-type or mutated glucose binding protein, e.g., from E. coli. (See the above-cited US 2003/0134346 for information regarding suitable mutated binding proteins.) For some applications, the concentration of the analyte is calculated by calculating a ratio of an emission level of the donor at a first wavelength to an emission level of the acceptor at a second wavelength (or vice versa).

For some applications in which the analyte includes glucose, the binding protein is a wild-type or mutated GLUT2 protein. Cells 26 are genetically engineered to overexpress the transmembranal GLUT2 protein. In some embodiments, overexpressing wild type GLUT2 helps bind a greater number of glucose molecules relative to wild type cells. Alternatively, cells 26 are engineered to overexpress a mutated GLUT2 (expressing the donor and acceptor moieties as described herein with reference to the GBP mutant), configured to measure glucose concentration in addition to inducing increased binding of glucose molecules by cells 26.

For some applications, techniques described herein are practiced using techniques described in one or more of the following references (the "selected references"): PCT Patent Publication WO 2006/006166 to Gross et al., United States Patent Application Publication 2005/0118726 to Schultz et al., United States Patent Application Publication 2003/0232370 to Trifiro, US Patent 5,998,204 to Tsien et al., and the above-cited articles to Ye K et al., Tolosa L et al., Hellinga H et al., and Fehr M et al.

For some applications, the fluorescent protein donor includes cyan fluorescent protein (CFP), and the fluorescent protein acceptor includes yellow fluorescent protein (YFP). Binding of glucose to a glucose binding protein induces a conformational change in the protein, thereby increasing the distance between the CFP and the YFP. To detect this change, light source 28 emits light at 430 nm, and a ratio of 535 nm and 480 nm wavelengths entering optical detector 30 is calculated in order to determine a level of glucose attachment at the binding proteins, using techniques known in the art (and described in references incorporated herein by reference, particularly the selected references described hereinabove). Alternatively or additionally, the protein comprises another donor/acceptor pair, such as blue fluorescent protein (BFP)/green fluorescent protein (GFP), GFP/Rhodamine, FITC/Cy3, FITC/Rhodamine, or another pair in which the donor emission spectrum overlaps the excitation spectrum of the acceptor. For some applications, the sensor protein includes one or more flexible hinge regions that enable the winding of large molecules, so as to enable energy transfer between the donor and the acceptor.

In some embodiments of the present invention, the GFP and the YFP sequences are ligated to respective ends of the glucose binding protein (GBP) sequence. It is to be noted, however, that the scope of the present invention includes gene fusion techniques other than those described explicitly herein. For example, the GFP and YFP sequences may be fused at respective sites within the GBP sequence itself. For some applications, placement of fluorescent proteins (e.g., GFP and/or YFP) within the GBP sequence enhances the ability of a FRET system to identify changes in glucose concentration.

In an embodiment of the present invention, the sensor protein is encoded by an isolated nucleic acid fragment having a nucleotide sequence represented by Sequence No. 1. For some applications, the sensor protein further comprises a leading peptide that directs the protein to the cell membrane, such as represented by Sequence No. 2, or, alternatively, a leading peptide that directs the cell to secrete the protein.

In some embodiments of the present invention, cells 26 express proteins which possess various binding affinities for the analyte. Typically, a first subset of cells 26 expresses proteins possessing a higher affinity for the analyte while a second subset of cells 26 expresses a protein possessing a lower affinity for the analyte. In some embodiments, one cell expresses various proteins, with respective binding affinities for the analyte. The proteins are genetically engineered such that the proteins possessing a high affinity to the analyte comprise a first fluorophore, (e.g., GFP) while the proteins possessing a lower affinity to the analyte comprise a second fluorophore (e.g., YFP). Typically, the first and second fluorophores fluoresce in response to respective excitation spectrums indicating physiological-to-low or physiological-to-high concentrations of the analyte, respectively. Alternatively, the first and second fluorophores fluoresce in response to different ranges of supraphysiological or subphysiological concentrations of the analyte. Typically, the cells are configured to overexpress the proteins possessing a high affinity to the analyte such that in the presence of low concentrations of the analyte, a majority of analyte will bind to the high-affinity proteins rather than to the low-affinity proteins, resulting in an intense emission from the first fluorophore. Thus, fluorescence emitted from the second fluorophore in response to excitation is indicative of the presence of a concentration of the analyte above a threshold concentration.

Membrane 42 and/or chamber 24 typically comprise at least one of: a hydrogel, an alginate (e.g., calcium alginate or sodium alginate), polyvinyl chloride acrylic copolymer, polylysine, chitosan, polyvinyl alcohol, polyethylene glycol, cellulose ester, cellulose acetate, a biocompatible polymer, silicone, and polyurethane. For some applications, membrane 42 comprises an anti-fibrosis factor and/or an anti-inflammatory factor (e.g., cortisone) and/or an angiogenic factor (e.g., VEGF). In an embodiment, cells 26 are engineered to be able to express the anti-inflammatory factor and/or the anti-fibrosis factor and/or the angiogenic factor, and membrane 42 allows the expressed factor to pass therethrough, in order to reduce a reaction of the subject's body to the implanted chamber.

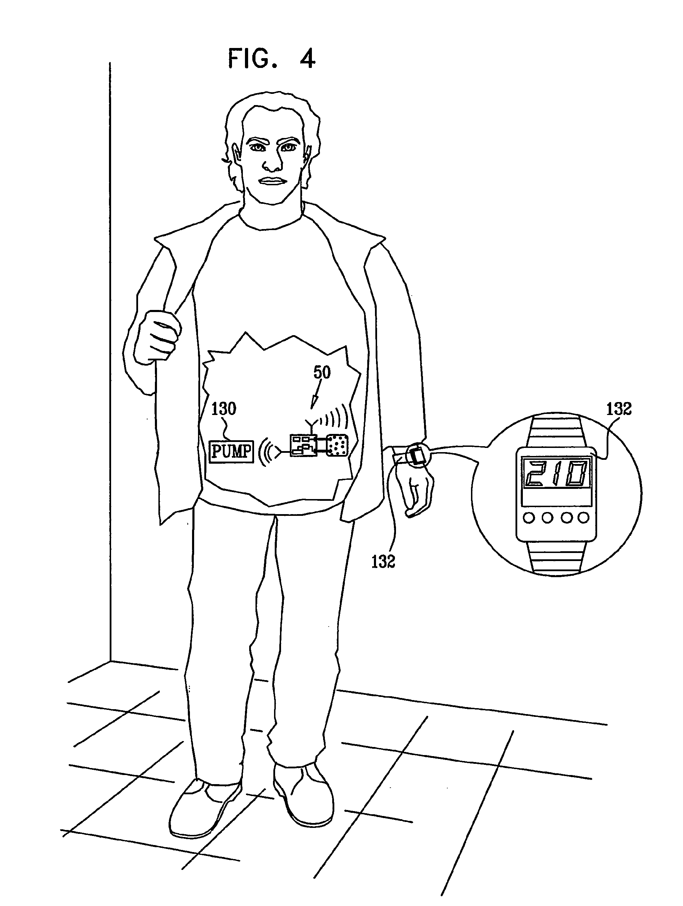

Reference is now made to Figs. 1-4. Fig. 4 is a schematic illustration of a system incorporating device 50 and other apparatus. (It is noted that devices 10 or 100 could replace device 50 in the embodiment shown in Fig. 4.) For some applications, device 50 comprises a transmitter 90, which transmits the collected data (typically wirelessly) to electronic receiving apparatus 132, such as a watch with receiving capabilities, a personal computer, a cell phone or a PDA. Receiving apparatus 132 is typically configured to present the data on a screen of the apparatus and/or to generate an alarm message (e.g., "Blood sugar too high (low)!"), as appropriate, for review by the patient, the patient's parent or caregiver, or a physician.

For some applications, device 50 transmits raw data to the receiving apparatus, and the receiving apparatus is programmed (in hardware or software) to perform all or a portion of the processing necessary to translate the raw data into concentration-indicating data. As appropriate, techniques described in one or more of the selected references may be used for this purpose. For some applications, the receiving apparatus is configured to transmit the raw data or concentration data over a public wireless or wired communication network. Alternatively or additionally, the receiving apparatus is coupled to a drug-delivery unit 130 such as an insulin pump, which supplies insulin or another drug to the body in response to the level of the analyte determined by the device.

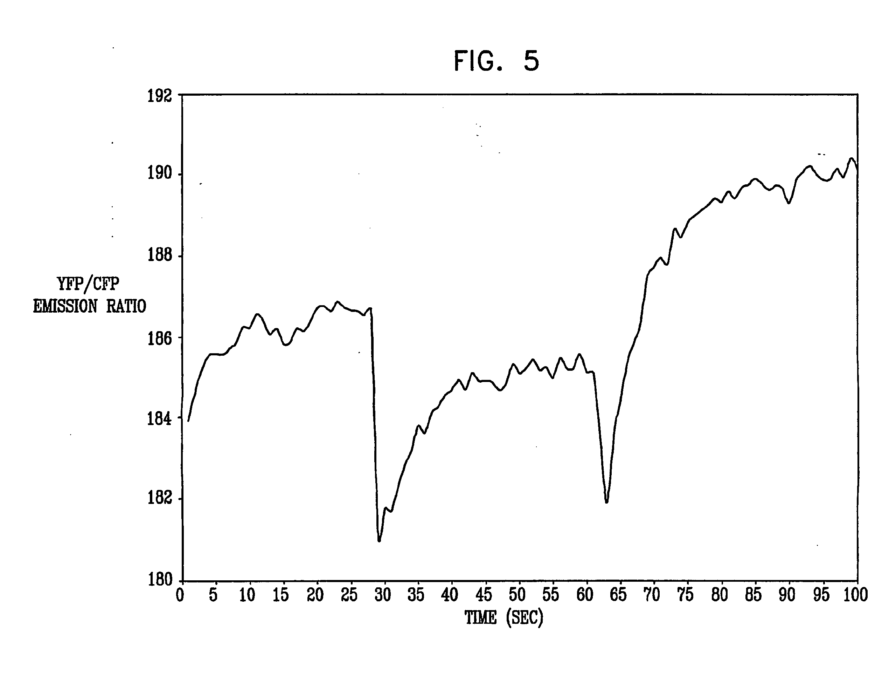

Fig. 5 is a graph showing experimental in vitro results obtained using genetically-engineered live cells.

For some applications, the concentration of the analyte is measured intermittently. Following each measurement, the likelihood of unbound analyte within chamber 24 is minimized due to application of agents or techniques which either reverse glucose binding to cells 26 and/or increase consumption of free analyte within chamber 24. Typically, entrance of new analyte into chamber 24 is minimized or eliminated during this period (e.g., with a mechanical inhibitor such as a very low molecular cutoff weight membrane, or a non-permeable barrier). Thus, basal levels of the analyte are attained prior to each successive measurement. In some embodiments, chamber 24 comprises cells characterized by their heightened metabolism of the analyte. For applications in which the analyte includes glucose, the cells may, for example, comprise algae. For other embodiments in which the analyte includes glucose, the cells are characterized by various consumption levels of glucose, e.g., mammalian cells such as COS7 or HepG2 cells. For some applications, the genetically engineered cells 26 themselves are characterized by high consumption rates of the analyte such that increased amounts of analyte are consumed by the cells, thus minimizing the amount of free analyte in chamber 24.

In some embodiments, an electrochemical method includes applying an electrical current to chamber 24 (either continuously or in pulses) which typically polarizes both the analyte as well as the protein configured for binding therewith. Such polarization repels the analyte from the protein so that the analyte may be passed through membrane 42 or be consumed by the cells, e.g., algae or mammalian cells, present in chamber 24. The electrochemical method may be used independently of or in combination with the analyte-consuming cells.

Construction of the Fusion Protein: The Enhanced Cyan Fluorescent Protein (ECFP) gene was amplified from pECFP-mem (Clontech) using forward and reverse primers that incorporated HindIII and EcoRII sites, respectively, and the amplicon was introduced into pEYFP-N1 (Clontech), creating pCY. A mglB (from E. coli K12) PCR product encoding mature glucose/galactose binding protein (GGBP) (positions 70-927 relative to ATG) was inserted into the EcoRI/BamHI sites of the plasmid pCY, generating pCGY (i.e., p-CFP-GGBP-YFP). The pCGY was transferred to COS-7 cells. The GGBP sequence was isolated from the bacteria *Escherichia coli.* The CFP and YFP sequences are from the jellyfish *Aequorea Victoria.* The "mem" (membrane) sequence is a sequence found in the mouse (*mus musculus*) genome. The "mem" sequence contains twenty amino acids from the N-terminus of the protein neuromodulin (GAP-43), which encodes for a posttranslational palmitoylation (covalent attachment of fatty acids to cysteine residues of membrane proteins) signal sequence which targets proteins to the plasma membrane. Fusing the "mem" sequence to the pCGY helps ensure migration to the plasma membrane of the genetically engineered sensing molecule.

COS7 (African green monkey kidney) cells were grown at 37°C in a humidified atmosphere with 5% CO2. Cell cultures were maintained in Dulbecco's modified Eagle's medium (DMEM) supplemented with 10% (v/v) fetal bovine serum (FBS) and penicillin/streptomycin (Biological Industries, Bet-Haemek, Israel). Cells were sub-cultured in Labtek glass coverslip chambers (Nalgene Nunc International, Rochester, NY). Cells were grown to between 50 and 60% confluence and then transfected with 0.5-1.5 ug DNA per Labtek chamber (8.4 cm2) using FuGENE-6 reagent (Roche Applied Science, Mannheim, Germany). Experiments and confocal laser-scanning microscopy images were obtained 35-40 hours after transfection.

Images were captured using the Zeiss LSM PASCAL, equipped with an Axiovert 200 inverted microscope. Argon 458 nm, 488 nm and 514 nm lasers were used for ECFP and EYFP measurements. Image capture was carried out using the advanced multi-time series macro set, with embedded reflection-mode autofocusing (Zeiss, Jena, Germany), in the presence of an electronic temperature-controlled air-stream incubator. Images were generated and analyzed using the Zeiss LSM software and NIH Image software (W. Rasband, NIH, Bethesda, MD). Images were acquired within the linear detection range of the camera at intervals of two or three seconds for up to five minutes. Glucose free Dulbecco's modified Eagle's medium was used to remove external glucose. Analyses were repeated at least four times with similar results.

In vitro characterization of pCGY: Glucose-dependent ratio changes were calculated, and are shown in Fig. 5. Glucose in different concentrations was added to a medium containing a single COS-7 cell prepared as described. At t ∼ 27 seconds, 1 mM glucose was added to the glucose-free medium, leading to a decrease in the YFP to CFP emission ratio within approximately two seconds. Following the addition and detection of glucose, external glucose was removed by perfusion with glucose-free solution. The subsequent increase in ratio over approximately 20 seconds indicates reversible glucose detection. Similar results are shown to have been obtained half a minute later, when 10 mM glucose was added to, and then removed from, the medium.

In an embodiment, cells are genetically engineered to express glucose oxidase (GOx) in a patient's body. For some applications, the GOx is secreted from the cells into the chamber. Some implantable glucose sensors that are known in the art use GOx to convert blood glucose into gluconic acid. The gluconic acid is converted into oxygen, and the oxygen concentration is measured amperometrically to determine the glucose concentration. The genetically engineered cells of this embodiment typically generate sufficient GOx to maintain an implantable blood glucose sensor for weeks or months. This embodiment may be practiced in combination with techniques described in the above-cited article by Scognamiglio et al.

Alternatively, the cells are engineered to express another enzyme used to convert glucose into gluconic acid.

Alternatively, the implanted cells are genetically engineered to express glucokinase without a catalytic domain, for use in an implantable glucose sensor. This embodiment may be practiced in combination with techniques described in the above-cited US Patent Application Publication 2003/0232370 to Trifiro and/or the above-cited article by Scognamiglio et al.

In accordance with an embodiment, cells 26 secrete two or more of: (a) a protein suitable for use in a FRET measurement, as described hereinabove, (b) GOx, for use as described hereinabove, and (c) glucokinase, for use as described hereinabove.

In an embodiment, a method comprises administering to a patient a drug to kill cells implanted in the patient. For example, the implanted cells may eventually cease to function for their intended purpose, and so may need to be replaced. (In addition, some implanted cells may, under certain circumstances, escape from a chamber containing them, and therefore need to be eliminated.) For some applications, the drug is administered to the patient when the patient is asymptomatic with respect to the implanted cells. For some applications, the drug is administered to kill the cells while they are in an implanted chamber. For some applications, the method further comprises implanting the cells in the patient prior to administering the drug.

For some applications, the drug is administered in conjunction with removing an implanted chamber from the patient's body (for example, to kill any cells that may escape during or prior to the removal procedure). Optionally, a new chamber is subsequently implanted. Alternatively, the implanted chamber is not removed from the patient's body, and the method comprises administering the drug to kill the cells in the chamber while it remains in the patient's body. For some applications, the drug includes a promoter, e.g., to control the expression of a suicide gene incorporated into a plasmid in the implanted cells. In an embodiment, the suicide gene includes thymidine kinase and the drug includes ganciclovir. Techniques may be incorporated that are described in the above-cited article by Fillat et al., which is incorporated herein by reference. In an embodiment, the drug is administered systemically (e.g., intramuscularly, intravenously, or orally), and travels to a site where the cells are located. Alternatively, the drug is administered directly to a site where the cells are located.

In an embodiment, a tetracycline-responsive promoter is used to regulate implanted cell activity (e.g., transiently increase or decrease sensor protein production), without killing the implanted cells. For some applications, techniques may be incorporated that are described in the above-cited article by Ackland-Berglund et al., which is incorporated herein by reference.

Typically, but not necessarily, the total volume of the implanted apparatus is between about 10 and about 50 cc, e.g., about 20-30 cc. For some applications, however, smaller or larger apparatus may also be used.

In an embodiment, a method comprises administering, to a subject, a promoter that regulates protein expression of cells implanted in the subject. For some applications, the cells are implanted for sensing a concentration of a blood constituent. For some applications, a level of expression of the sensor protein described hereinabove is regulated, such as to optimize the FRET measurement. For some applications, the promoter is selected to reduce protein expression, for example, if the subject has a reaction to the protein.

In an embodiment of the present invention, a glucose sensor is adapted to be implanted in cerebral spinal fluid (CSF) of the spinal cord. Because the constituents of CSF are more tightly controlled than those of blood, there is generally less background noise in CSF that might reduce the accuracy of the sensor. For some applications, the techniques of this embodiment are practiced in conjunction with the glucose sensing techniques described herein, *mutatis mutandis.* Alternatively, these techniques are practiced in conjunction with implantable glucose sensors known in the art, *mutatis mutandis.*

In an embodiment, a method comprises implanting an active medical device inside bone, and detecting or affecting a property of blood or another body fluid in fluid communication with the medical device. The lack of fibrosis inside bone generally results in good fluid communication between the medical device and the blood. For some applications, the medical device comprises a glucose sensor. For some applications, the bone includes bone of a tooth or bone of a long bone.

In an embodiment, implantable cells are genetically engineered to express a promoter that is induced in general, but which is no longer induced when a substance is administered to a body of a subject in which the cells are implanted. For some applications, the inducing substance includes an antibiotic. Typically, the promoter is capable of activating and/or deactivating one or more genes of interest. In this manner, for example, administration of an antibiotic can be used to transiently turn off the activity of cells 26.

For some applications, glucose measuring techniques described herein are combined with intermittent blood glucose tests (e.g., weekly or monthly blood tests), in order to recalibrate the implanted apparatus.

It will be appreciated by persons skilled in the art that the present invention is not limited to detecting blood glucose, but that blood glucose is used by way of example. The scope of the present invention includes determining and/or monitoring levels of other analytes in the body.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus (50) for detecting a concentration of an analyte in a subject, comprising:
an implantable chamber (24) that comprises live cells (26) that are genetically engineered to produce, in a subject's body, a sensor protein comprising a fluorescent protein donor, a fluorescent protein acceptor and a binding protein for the analyte; and
an implantable sensor (22) adapted to sense a characteristic of the protein and to generate a signal, in response to the sensed characteristic, that is indicative of a physiological parameter of a subject, the sensor (22) including a light source (28) and an optical detector (30) adapted to detect a level of fluorescence resonance energy transfer (FRET);
**characterized in that** the sensor (22) further comprises a signal generator (86) for driving the light source (28), and a signal processor (88) for receiving a signal from the optical detector (30); and **in that** the apparatus further comprises:
a set of one or more optical fibers (70) coupled between the light source (28) and the chamber (24) to enable the light source (28) to illuminate the cells (26), and a set of optical fibres (72) for carrying light from chamber (24) to the signal processor (88) via the optical detector (30); or
a common set of one or more optical fibres oonfigured to operate bidirectionally, carrying light from the light source (28) to the chamber (24), and carrying modified light ftom the chamber (24) back to the sensor (22).

2. The apparatus (50) according to claim 1, further comprising a housing comprising the sensor (22), wherein the sensor (22) further comprises a transmitter (90), and wherein the cells are not disposed within the housing.

3. The apparatus (50) according to claim 2, wherein the housing is configured to be implanted at a first site of the subject, and the implantable chamber (24) is configured to be implanted at a second site of the subject 3-20 cm from the first site.

4. The apparatus (50) according to claim 1, wherein the cells (26) are genetically engineered to secrete a factor selected from the group consisting of: an anti-fibrosis factor and an anti-inflammatory factor, and wherein the apparatus is configured to allow passage out of the apparatus of the selected factor.

5. The apparatus (50) according to any one of claims 1-4, wherein the cells (26) are genetically engineered to secrete an angiogenic factor, and wherein the apparatus is configured to allow passage out of the apparatus of the angiogenic factor.

6. The apparatus (50) according to claim 5, wherein the angiogenic factor comprises vascular endothelial growth factor (VEGF).

7. The apparatus (50) according to any one of claims 1-6,
wherein the chamber comprises a mechanical inhibitor configured to facilitate intermittent measurement of an analyte by minimizing entrance of the analyte into the chamber,
wherein, responsively to the intermittent measurement, the sensor (22) is configured to determine an extent of binding of the analyte to the protein,
wherein the apparatus, by the mechanical inhibitor, is configured to intermittently establish a basal level of binding of the analyte to the protein by reducing binding of the analyte to the protein, and
wherein the sensor (22) is configured to calibrate the determining of the extent of binding by measuring the basal level of binding.

8. The apparatus (50) according to claim 1, wherein the physiological parameter of the subject includes a concentration of glucose, and wherein the live cells (26) are genetically engineered to produce GLUT2.

9. The apparatus (50) according to claim 1, wherein the live cells (26) are genetically engineered to produce first and second proteins, the first and second proteins having different respective affinities for an analyte, and wherein the implantable sensor is configured to determine the physiological parameter by determining an extent of binding of the analyte to the first and second proteins.

10. The apparatus (50) according to claim 8, wherein the live cells are genetically engineered to overexpress GLUT2.

## Patentansprüche

1. Vorrichtung (50) zum Erkennen einer Konzentration eines Analyten in einem Subjekt, die Folgendes umfasst:
eine implantierbare Kammer (24), die gentechnisch hergestellte lebende Zellen (26) enthält, um im Körper eines Subjekts ein Sensorprotein zu erzeugen, das einen fluoreszierenden Proteinspender, einen fluoreszierenden Proteinakzeptor und ein Bindungsprotein für den Analyt umfasst; und
einen implantierbaren Sensor (22) zum Erfassen einer Charakteristik des Proteins und zum Erzeugen eines Signals als Reaktion auf die erfasste Charakteristik, das einen physiologischen Parameter eines Subjekts anzeigt, wobei der Sensor (22) eine Lichtquelle (28) und einen optischen Detektor (30) zum Erkennen eines Niveaus an Fluoreszenz-Resonanz-Energie-Transfer (FRET) beinhaltet;
**dadurch gekennzeichnet, dass** der Sensor (22) ferner einen Signalgenerator (86) zum Ansteuern der Lichtquelle (28) und einen Signalprozessor (88) zum Empfangen eines Signals von dem optischen Detektor (30) umfasst; und dadurch, dass die Vorrichtung ferner Folgendes umfasst:
einen Satz von einer oder mehreren optischen Fasern (70), die zwischen der Lichtquelle (28) und der Kammer (24) gekoppelt sind, um es zu ermöglichen, dass die Lichtquelle (28) die Zellen (26) beleuchtet, und einen Satz von optischen Fasern (72) zum Führen von Licht von der Kammer (24) zum Signalprozessor (88) über den optischen Detektor (30); oder
einen gemeinsamen Satz von einer oder mehreren optischen Fasern, die zum bidirektionalen Arbeiten konfiguriert sind und Licht von der Lichtquelle (28) zu der Kammer (24) führen und modifiziertes Licht von der Kammer (24) zurück zum Sensor (22) führen.

2. Vorrichtung (50) nach Anspruch 1, die ferner ein Gehäuse mit dem Sensor (22) umfasst, wobei der Sensor (22) ferner einen Sender (90) umfasst und wobei sich die Zellen nicht in dem Gehäuse befinden.

3. Vorrichtung (50) nach Anspruch 2, wobei das Gehäuse zum Implantieren an einem ersten Ort in dem Subjekt konfiguriert ist und die implantierbare Kammer (24) zum Implantieren an einem zweiten Ort in dem Subjekt 3-20 cm entfernt von dem ersten Ort konfiguriert ist.

4. Vorrichtung (50) nach Anspruch 1, wobei die Zellen (26) gentechnisch hergestellt werden, um einen Faktor zu sekretieren, der ausgewählt ist aus der Gruppe bestehend aus einem antifibrotischen Faktor und einem entzündungshemmenden Faktor, und wobei die Vorrichtung so konfiguriert ist, dass sie ein Austreten des gewählten Faktors aus der Vorrichtung zulässt.

5. Vorrichtung (50) nach einem der Ansprüche 1-4, wobei die Zellen (26) gentechnisch hergestellt werden, um einen angiogenen Faktor zu sekretieren, und wobei die Vorrichtung so konfiguriert ist, dass sie ein Austreten des angiogenen Faktors aus der Vorrichtung zulässt.

6. Vorrichtung (50) nach Anspruch 5, wobei der angiogene Faktor vaskulären endothelialen Wachstumsfaktor (VEGF) umfasst.

7. Vorrichtung (50) nach einem der Ansprüche 1-6,
wobei die Kammer einen mechanischen Inhibitor umfasst, der zum Erleichtern einer intermittierenden Messung eines Analyten durch Minimieren des Eintretens des Analyten in die Kammer konfiguriert ist,
wobei der Sensor (22) als Reaktion auf die intermittierende Messung zum Ermitteln eines Ausmaßes an Bindung des Analyten an das Protein konfiguriert ist,
wobei die Vorrichtung, durch den mechanischen Inhibitor, zum intermittierenden Herstellen eines basalen Bindungsniveaus des Analyten an das Protein durch Reduzieren der Bindung des Analyten an das Protein konfiguriert ist, und
wobei der Sensor (22) zum Kalibrieren des Ermittelns des Ausmaßes an Bindung durch Messen des basalen Bindungsniveaus konfiguriert ist.

8. Vorrichtung (50) nach Anspruch 1, wobei der physiologische Parameter des Subjekts eine Glukosekonzentration beinhaltet und wobei die lebenden Zellen (26) gentechnisch hergestellt werden, um GLUT2 zu erzeugen.

9. Vorrichtung (50) nach Anspruch 1, wobei die lebenden Zellen (26) gentechnisch hergestellt werden, um erste und zweite Proteine zu erzeugen, wobei die ersten und zweiten Proteine unterschiedliche jeweilige Affinitäten für einen Analyt aufweisen und wobei der implantierbare Sensor zum Ermitteln des physiologischen Parameters durch Ermitteln eines Ausmaßes an Bindung des Analyten an das erste und zweite Protein konfiguriert ist.

10. Vorrichtung (50) nach Anspruch 8, wobei die lebenden Zellen gentechnisch hergestellt werden, um GLUT2 zu überexprimieren.

## Revendications

1. Dispositif (50) pour la détection d'une concentration en un analyte chez un sujet, qui comprend :
une chambre implantable (24) qui comprend des cellules vivantes (26) génétiquement modifiées pour produire, dans le corps d'un sujet, une protéine « senseur » comprenant un donneur protéique fluorescent, un accepteur protéique fluorescent et une protéine de liaison à l'analyte ; et
un capteur implantable (22) adapté pour reconnaître une caractéristique de la protéine et générer, en réponse à la caractéristique reconnue, un signal qui est indicateur d'un paramètre physiologique du sujet, le capteur (22) incluant une source lumineuse (28) et un détecteur optique (30) adapté pour détecter un niveau de transfert d'énergie de résonance par fluorescence (FRET) ;
**caractérisé en ce que** le capteur (22) comprend aussi un générateur de signal (86) pour actionner la source lumineuse (28) et un système de traitement du signal (88) pour recevoir un signal à partir du détecteur optique (30) ; et **en ce que** le dispositif comprend également :
un jeu d'une ou de plusieurs fibres optiques (70) raccordé entre la source lumineuse (28) et la chambre (24) pour permettre à la source lumineuse (28) d'illuminer les cellules (26) et un jeu de fibres optiques (72) pour transporter la lumière de la chambre (24) au système de traitement du signal (88) par le biais du détecteur optique (30) ; ou
un jeu commun d'une ou de plusieurs fibres optiques configurées pour fonctionner de manière bidirectionnelle, en transportant la lumière de la source lumineuse (28) à la chambre (24) et en ramenant la lumière modifiée de la chambre (24) au capteur (22).

2. Dispositif (50) selon la revendication 1, qui comprend également un boîtier abritant le capteur (22), le capteur (22) comprenant également un transmetteur (90) et les cellules n'étant pas contenues à l'intérieur du boîtier.

3. Dispositif (50) selon la revendication 2, le boîtier étant configuré pour être implanté en un premier site chez le sujet, la chambre implantable (24) étant configurée pour être implantée en un second site chez le sujet à 3-20 cm du premier site.

4. Dispositif (50) selon la revendication 1, les cellules (26) étant génétiquement modifiées pour sécréter un facteur sélectionné dans le groupe consistant en un facteur antifibrose et un facteur anti-inflammatoire, le dispositif étant configuré pour permettre un passage du facteur sélectionné en dehors du dispositif.

5. Dispositif (50) selon l'une quelconque des revendications 1-4, les cellules (26) étant génétiquement modifiées pour sécréter un facteur angiogène, le dispositif étant configuré pour permettre un passage du facteur angiogène en dehors du dispositif.

6. Dispositif (50) selon la revendication 5, le facteur angiogène comprenant le facteur de croissance endothélial vasculaire (VEGF).

7. Dispositif (50) selon l'une quelconque des revendications 1-6,
la chambre comprenant un inhibiteur mécanique configuré pour faciliter la mesure intermittente d'un analyte en minimisant l'entrée de l'analyte dans la chambre,
le capteur (22) étant configuré pour déterminer, en réponse à la mesure intermittente, le niveau de liaison de l'analyte à la protéine,
le dispositif étant configuré, par le biais de l'inhibiteur mécanique, pour établir de manière intermittente un taux de base de liaison de l'analyte à la protéine en réduisant la liaison de l'analyte à la protéine,
le capteur (22) étant configuré pour calibrer la détermination du niveau de liaison en mesurant le taux de liaison de base.

8. Dispositif (50) selon la revendication 1, le paramètre physiologique du sujet incluant une concentration en glucose, les cellules vivantes (26) étant génétiquement modifiées pour produire le GLUT2.

9. Dispositif (50) selon la revendication 1, les cellules vivantes (26) étant génétiquement modifiées pour produire une première et une seconde protéines, les première et seconde protéines ayant des affinités respectives différentes pour un analyte, le capteur implantable étant configuré pour déterminer le paramètre physiologique par une évaluation d'un niveau de liaison de l'analyte aux première et seconde protéines.

10. Dispositif (50) selon la revendication 8, les cellules vivantes étant génétiquement modifiées pour surexprimer le GLUT2.


### SEQUENCE LISTING

<110> Glusense, Ltd.
<120> Implantable sensor
<130> P8322.EPP-08/PJE:sgh
<140> 07736139.2
   <141> 2007-03-28
<160> 2
<170> PatentIn version 3.5
<210> 1
   <211> 2313
   <212> DNA
   <213> Artificial Sequence
<220>
   <223> artificial sequence comprising coding sequences from the following organisms: Aequorea victoria and Escherichia coli K12, and linker sequences comprising: poly-Gly and restriction sites
<220>
   <221> CDS
   <222> (1)..(2313)
<400> 1
   atg gtg agc aag ggc gag gag ctg ttc acc ggg gtg gtg ccc atc ctg 48
   gtc gag ctg gac ggc gac gta aac ggc cac aag ttc agc gtg tcc ggc 96
   gag ggc gag ggc gat gcc acc tac ggc aag ctg acc ctg aag ttc atc 144
   tgc acc acc ggc aag ctg ccc gtg ccc tgg ccc acc ctc gtg acc acc 192
   ctg acc tgg ggc gtg cag tgc ttc agc cgc tac ccc gac cac atg aag 240
   cag cac gac ttc ttc aag tcc gcc atg ccc gaa ggc tac gtc cag gag 288
   cgc acc atc ttc ttc aag gac gac ggc aac tac aag acc cgc gcc gag 336
   gtg aag ttc gag ggc gac acc ctg gtg aac cgc atc gag ctg aag ggc 384
   atc gac ttc aag gag gac ggc aac atc ctg ggg cac aag ctg gag tac 432
   aac tac atc agc cac aac gtc tat atc acc gcc gac aag cag aag aac 480
   ggc atc aag gcc aac ttc aag atc cgc cac aac atc gag gac ggc agc 528
   gtg cag ctc gcc gac cac tac cag cag aac acc ccc atc ggc gac ggc 576
   ccc gtg ctg ctg ccc gac aac cac tac ctg agc acc cag tcc gcc ctg 624
   agc aaa gac ccc aac gag aag cgc gat cac atg gtc ctg ctg gag ttc 672
   gtg acc gcc gcc ggg atc act ctc ggc atg gac gag ctg tac aag ggt 720
   ggt ggt gct gat act cgc att ggt gta aca atc tat aag tac gac gat 768
   aac ttt atg tct gta gtg cgc aag gct att gag caa gat gcg aaa gcc 816
   gcg cca gat gtt cag ctg ctg atg aat gat tct cag aat gac cag tcc 864
   aag cag aac gat cag atc gac gta ttg ctg gcg aaa ggg gtg aag gca 912
   ctg gca atc aac ctg gtt gac ccg gca gct gcg ggt acg gtg att gag 960
   aaa gcg cgt ggg caa aac gtg ccg gtg gtt ttc ttc aac aaa gaa ccg 1008
   tct cgt aag gcg ctg gat agc tac gac aaa gcc tac tac gtt ggc act 1056
   gac tcc aaa gag tcc ggc att att caa ggc gat ttg att gct aaa cac 1104
   tgg gcg gcg aat cag ggt tgg gat ctg aac aaa gac ggt cag att cag 1152
   ttc gta ctg ctg aaa ggt gaa ccg ggc cat ccg gat gca gaa gca cgt 1200
   acc act tac gtg att aaa gaa ttg aac gat aaa ggc atc aaa act gaa 1248
   cag tta cag tta gat acc gca atg tgg gac acc gct cag gcg aaa gat 1296
   aag atg gac gcc tgg ctg tct ggc ccg aac gcc aac aaa atc gaa gtg 1344
   gtt atc gcc aac aac gat gcg atg gca atg ggc gcg gtt gaa gcg ctg 1392
   aaa gca cac aac aag tcc agc att ccg gtg ttt ggc gtc gat gcg ctg 1440
   cca gaa gcg ctg gcg ctg gtg aaa tcc ggt gca ctg gcg ggc acc gta 1488
   ctg aac gat gct aac aac cag gcg aaa gcg acc ttt gat ctg gcg aaa 1536
   aac ctg gcc gat ggt aaa ggt gcg gct gat ggc acc aac tgg aaa atc 1584
   ggt ggt ggt atg gtg agc aag ggc gag gag ctg ttc acc ggg gtg gtg 1632
   ccc atc ctg gtc gag ctg gac ggc gac gta aac ggc cac aag ttc agc 1680
   gtg tcc ggc gag ggc gag ggc gat gcc acc tac ggc aag ctg acc ctg 1728
   aag ttc atc tgc acc acc ggc aag ctg ccc gtg ccc tgg ccc acc ctc 1776
   gtg acc acc ttc ggc tac ggc ctg cag tgc ttc gcc cgc tac ccc gac 1824
   cac atg aag cag cac gac ttc ttc aag tcc gcc atg ccc gaa ggc tac 1872
   gtc cag gag cgc acc atc ttc ttc aag gac gac ggc aac tac aag acc 1920
   cgc gcc gag gtg aag ttc gag ggc gac acc ctg gtg aac cgc atc gag 1968
   ctg aag ggc atc gac ttc aag gag gac ggc aac atc ctg ggg cac aag 2016
   ctg gag tac aac tac aac agc cac aac gtc tat atc atg gcc gac aag 2064
   cag aag aac ggc atc aag gtg aac ttc aag atc cgc cac aac atc gag 2112
   gac ggc agc gtg cag ctc gcc gac cac tac cag cag aac acc ccc atc 2160
   ggc gac ggc ccc gtg ctg ctg ccc gac aac cac tac ctg agc tac cag 2208
   tcc gcc ctg agc aaa gac ccc aac gag aag cgc gat cac atg gtc ctg 2256
   ctg gag ttc gtg acc gcc gcc ggg atc act ctc ggc atg gac gag ctg 2304
   tac aag taa 2313
<210> 2
   <211> 2376
   <212> DNA
   <213> Artificial sequence
<220>
   <223> Artificial sequence comprising coding sequences from the following organisms: Aequorea victoria, Escherichia coli K12, and Mus musculus, and contains linker sequences comprising restriction sites and poly-Gly
<220>
   <221> CDS
   <222> (1)..(2376)
<400> 2
   acc atg ctg tgc tgt atg aga aga acc aaa cag gtt gaa aag aat gat      48
   gag gac caa aag atc atg gtg agc aag ggc gag gag ctg ttc acc ggg      96
   gtg gtg ccc atc ctg gtc gag ctg gac ggc gac gta aac ggc cac aag     144
   ttc agc gtg tcc ggc gag ggc gag ggc gat gcc acc tac ggc aag ctg     192
   acc ctg aag ttc atc tgc acc acc ggc aag ctg ccc gtg ccc tgg ccc     240
   acc ctc gtg acc acc ctg acc tgg ggc gtg cag tgc ttc agc cgc tac     288
   ccc gac cac atg aag cag cac gac ttc ttc aag tcc gcc atg ccc gaa     336
   ggc tac gtc cag gag cgc acc atc ttc ttc aag gac gac ggc aac tac     384
   aag acc cgc gcc gag gtg aag ttc gag ggc gac acc ctg gtg aac cgc     432
   atc gag ctg aag ggc atc gac ttc aag gag gac ggc aac atc ctg ggg     480
   cac aag ctg gag tac aac tac atc agc cac aac gtc tat atc acc gcc     528
   gac aag cag aag aac ggc atc aag gcc aac ttc aag atc cgc cac aac     576
   atc gag gac ggc agc gtg cag ctc gcc gac cac tac cag cag aac acc     624
   ccc atc ggc gac ggc ccc gtg ctg ctg ccc gac aac cac tac ctg agc     672
   acc cag tcc gcc ctg agc aaa gac ccc aac gag aag cgc gat cac atg     720
   gtc ctg ctg gag ttc gtg acc gcc gcc ggg atc act ctc ggc atg gac     768
   gag ctg tac aag ggt ggt ggt gct gat act cgc att ggt gta aca atc     816
   tat aag tac gac gat aac ttt atg tct gta gtg cgc aag gct att gag     864
   caa gat gcg aaa gcc gcg cca gat gtt cag ctg ctg atg aat gat tct     912
   cag aat gac cag tcc aag cag aac gat cag atc gac gta ttg ctg gcg     960
   aaa ggg gtg aag gca ctg gca atc aac ctg gtt gac ccg gca gct gcg    1008
   ggt acg gtg att gag aaa gcg cgt ggg caa aac gtg ccg gtg gtt ttc    1056
   ttc aac aaa gaa ccg tct cgt aag gcg ctg gat agc tac gac aaa gcc    1104
   tac tac gtt ggc act gac tcc aaa gag tcc ggc att att caa ggc gat    1152
   ttg att gct aaa cac tgg gcg gcg aat cag ggt tgg gat ctg aac aaa    1200
   gac ggt cag att cag ttc gta ctg ctg aaa ggt gaa ccg ggc cat ccg    1248
   gat gca gaa gca cgt acc act tac gtg att aaa gaa ttg aac gat aaa 1296
   ggc atc aaa act gaa cag tta cag tta gat acc gca atg tgg gac acc 1344
   gct cag gcg aaa gat aag atg gac gcc tgg ctg tct ggc ccg aac gcc 1392
   aac aaa atc gaa gtg gtt atc gcc aac aac gat gcg atg gca atg ggc 1440
   gcg gtt gaa gcg ctg aaa gca cac aac aag tcc agc att ccg gtg ttt 1488
   ggc gtc gat gcg ctg cca gaa gcg ctg gcg ctg gtg aaa tcc ggt gca 1536
   ctg gcg ggc acc gta ctg aac gat gct aac aac cag gcg aaa gcg acc 1584
   ttt gat ctg gcg aaa aac ctg gcc gat ggt aaa ggt gcg gct gat ggc 1632
   acc aac tgg aaa atc ggt ggt ggt atg gtg agc aag ggc gag gag ctg 1680
   ttc acc ggg gtg gtg ccc atc ctg gtc gag ctg gac ggc gac gta aac 1728
   ggc cac aag ttc agc gtg tcc ggc gag ggc gag ggc gat gcc acc tac 1776
   ggc aag ctg acc ctg aag ttc atc tgc acc acc ggc aag ctg ccc gtg 1824
   ccc tgg ccc acc ctc gtg acc acc ttc ggc tac ggc ctg cag tgc ttc 1872
   gcc cgc tac ccc gac cac atg aag cag cac gac ttc ttc aag tcc gcc 1920
   atg ccc gaa ggc tac gtc cag gag cgc acc atc ttc ttc aag gac gac 1968
   ggc aac tac aag acc cgc gcc gag gtg aag ttc gag ggc gac acc ctg 2016
   gtg aac cgc atc gag ctg aag ggc atc gac ttc aag gag gac ggc aac 2064
   atc ctg ggg cac aag ctg gag tac aac tac aac agc cac aac gtc tat 2112
   atc atg gcc gac aag cag aag aac ggc atc aag gtg aac ttc aag atc 2160
   cgc cac aac atc gag gac ggc agc gtg cag ctc gcc gac cac tac cag 2208
   cag aac acc ccc atc ggc gac ggc ccc gtg ctg ctg ccc gac aac cac 2256
   tac ctg agc tac cag tcc gcc ctg agc aaa gac ccc aac gag aag cgc 2304
   gat cac atg gtc ctg ctg gag ttc gtg acc gcc gcc ggg atc act ctc 2352
   ggc atg gac gag ctg tac aag taa 2376